# EUROPEAN PATENT APPLICATION

(11) **EP 1 240 911 A1**
(43) Date of publication of application: **18.09.2002**
(21) Application number: 02005744.4
(22) Date of filing: 13.03.2002
(51) Int. Cl.: A61M 3/02

(54) **Intestinal treatment apparatus for self-use**

(30) Priority: 15.03.2001 IT MI010550
(71) Applicant: Di Cecco, Enio Riccardo, 20147 Milan (IT)
(72) Inventor: Di Cecco, Enio Riccardo, 20147 Milan (IT)
(74) Representative: Coloberti, Luigi

(57) **Abstract**

An intestinal treatment apparatus for self-use.

The apparatus comprises a seat (11) which can be fitted onto a toilet bowl (10) , provided on the rear portion with a duct (13) for feeding an intestinal lavage or treatment fluid; an injection speculum (15) for injecting the lavage fluid, is upwardly protruding inside the toilet bowl (10), to penetrate the anal tract of the intestine by a self-insertion action. The speculum (15) is of disposable type, and is removably connected to an end fitting (14) of the feeding conduit (13) or to a removable tubular element (17) forming an extension of the feed duct (13). A one-way valve (18) prevents the back flow of the organic substance towards the feed duct (13), while a pressure regulator (20) prevents accidental injection of strong jets of fluid during use. The apparatus may also be provided with a dispenser (28) for a curative substance which is mixed into the lavage fluid fed to the injection speculum (15).

## Description

### FIELD OF THE INVENTION

This invention relates to an apparatus of self-use type for intestinal treatment and cures, designed to allow, in a whole independent way, a lavage of the terminal portion of the intestine, or "colon", without any intervention by an operator.

In particular, the invention is directed to an apparatus of the aforementioned kind, by means of which it is possible to carry out an effective lavage action of the colon, in order to remove and evacuate the faecal contents stagnating in the latter, without the patient suffering any trauma, or having to leave home and go to medical centres appropriately specialised and equipped to perform such intestinal treatment of cleansing and cure.

### STATE OF THE ART

As is known, the colon hydrotherapy is a well known technique which has been practised for a long time for removing the faecal contents which tend to stagnate and adhere to the walls of the intestine, and which can cause the arising of functional alterations or diseases, such as cancer of the colon, the incidence of which on the male population proves to be extremely high, as it has been ascertained by university and clinical studies carried out both in Italy and abroad. In short, hydrotherapy of the colon, as a possible prevention against colon-rectal cancer, or treatment suitable for restoring the correct intestinal function, is performed by injecting a lavage fluid, such as water, in case mixed with a curative substance, into the intestine of a patient. The gentle and controlled injection of the lavage fluid is carried out by means of a special apparatus controlled by an operator, in order to soften and dissolve the intestinal contents which are then evacuated directly by means of the same apparatus, while maintaining the patient in a supine position on a special bed.

Even though this therapeutic technique has been known and practised for a long time, use is currently made of particularly gentle, highly complex and expensive equipment, made available in appropriately equipped hospitals or medical centres.

The complexity of the equipment and the difficulties which have in fact been encountered, have highly conditioned the use of this therapy which, on the contrary, has proven to be particularly effective in the cure and/or in the prevention of several pathologies.

In addition to the natural confidentiality which a treatment of this kind requires, in the case of patients who for various reasons require frequent lavage treatments, having to go to an equipped medical centre, in addition to consuming a considerable amount of time, also involves considerable costs.

### OBJECTS OF THE INVENTION

The main object of this invention is to provide an apparatus for performing intestinal lavages and cures of the self-use type, which can be used wholly independently by any patient, in his own home, without having to go to specially equipped medical centres.

A particular object of this invention is to provide an apparatus of the aforementioned kind, which is structurally simple and inexpensive, as well as being easy to install and to use on any toilet bowl.

A further object of this invention is to provide an apparatus of the aforementioned kind, which can be used wholly independently by any patient, without the assistance or presence of a specialised operator or personnel.

A still further object is to provide an apparatus for performing intestinal lavages and cures, which can be used with the utmost promptness and practicality, without any inconvenience, while maintaining extremely high hygienic conditions during use.

The absolute hygienic quality of the apparatus and the possibility of carrying out a thorough cleansing, enables an effective prevention and curative action both of the intestine and the vagina, by means of suitable medicinal preparations.

A still further object is to provide an apparatus of the aforesaid kind, designed to form an integral part of a toilet seat, while also permitting the normal use of the toilet bowl.

The apparatus has been specially designed for personal use, resulting available at any time and for any occurrence.

### BRIEF DESCRIPTION OF THE INVENTION

The above can be achieved by means of an apparatus of self-use type, for intestinal lavages and the like, according to claim 1.

More in particular, according to the invention an apparatus for intestinal lavages and the like has been provided, particularly suitable for use on a toilet bowl, characterized by comprising:
- a shaped seat for a toilet bowl, having a rear side and an inner edge;
- a feed duct for feeding a lavage fluid, said feeding duct extending at the rear side of the seat, said feeding duct comprising an end fitting at the rear side of the seat;
- a disposable injection speculum, or probe, for injecting a lavage fluid, removably connected to the end fitting of the feed duct, said speculum having a portion which extends upwards from the inside of the toilet bowl, for a length sufficient to penetrate the anal tract of the intestine;
- manually actuable flow control valve for adjusting the flow rate and for connection to a lavage fluid source; and
- manually actuable pressure control valve for controlling the pressure of the lavage fluid fed to the injection speculum.

According to a further feature, the apparatus can be provided with means for feeding a curative substance into the lavage fluid fed to the injection speculum or probe.

### BRIEF DESCRIPTION OF THE DRAWINGS

These and further features and advantages of an apparatus for intestinal lavages according to the invention, will be more clearly evident from the following description, with reference to the examples of the accompanying drawings, in which:
Figure 1 is a schematic view of a first embodiment of the apparatus according to the invention;
Fig. 2 is an enlarged cross-sectional view, along the line 2-2 of Fig. 1;
Fig. 3 shows a second embodiment of an apparatus according to the invention;
Fig. 4 is an enlarged sectional view according to Fig. 2, showing a variation of the injection speculum;
Fig. 5 is an enlarged cross-sectional view of the front end of the injection speculum;
Fig. 6 shows an enlarged view of a tubular member for connection of a flexible probe.
Fig. 7 is a schematic view of a further embodiment of the apparatus according to the invention;
Fig. 8 is an enlarged cross-sectional view according to line 8-8 of Fig. 7;
Fig. 9 is an enlarged detail of Fig. 7.

### DETAILED DESCRIPTION OF THE INVENTION

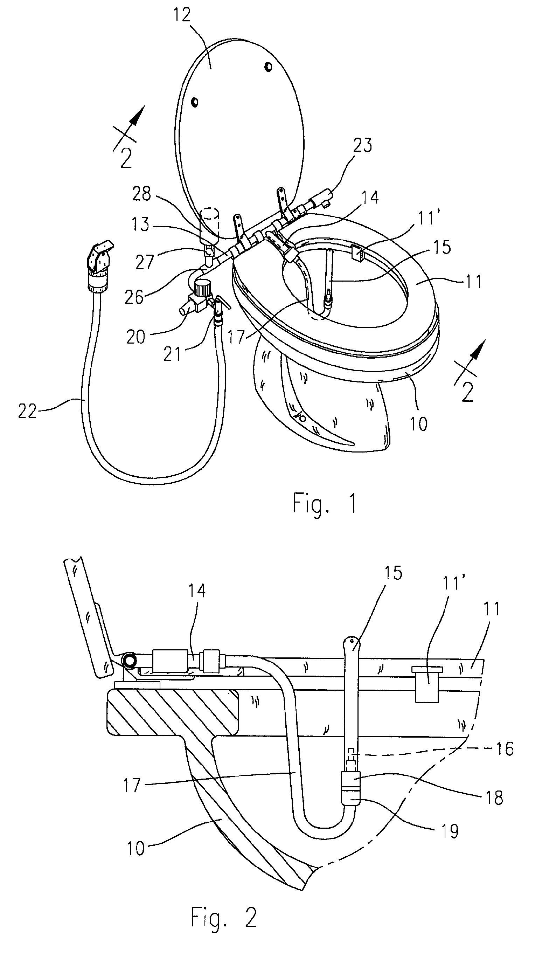

Hereunder a description is given of the general features of an apparatus according to the invention, with reference to the example of Figs. 1 and 2 of the accompanying drawings.

In Fig. 1, reference number 10 indicates a generic toilet bowl provided with an apparatus for intestinal lavages, suitable for independent use, according to this invention.

The apparatus substantially comprises an annularly shaped seat 11 having a central aperture designed to be rested on the bowl 10, provided with a lift-up lid 12, both hinged to the bowl 10.

The seat 11 in turn is provided, on the rear side, with a duct 13 which extends transversally and which ends with a pipe fitting 14 extending towards the inner edge of the seat 11; a speculum 15 of the disposable type, is removably secured to a V shaped pipe member 17, downwardly protruding into the bowl, and upwards.

In the example of Figs. 1 and 2, the speculum 15 is of the rigid type and substantially consists of a linear tubular element made of plastic material, having perforations at the closed tip, designed to be removably secured at the end 16 of the pipe member 17 which constitutes an extension of the pipe fitting 14.

The speculum 15 is vertically oriented upwards from the inside of the bowl 10, through the seat 11, ending at the same level or extending by a short length, for example by 5-6 cm, beyond the upper surface of the same seat so as to partially self-penetrate into the anal portion of the intestine, by merely sitting down.

The speculum 15 may comprise or be provided with a one-way check valve 18 at the rear end for connection to the pipe member 17; a ball joint 19 enables the end 16 of the pipe 17 and consequently the same speculum 15 to be angularly orientated in order to compensate for any movement of a patient resting on the seat 11 during an intestinal lavage treatment.

To prevent the speculum 15 and the check valve 18 from being re-used after a lavage treatment, and in order to ensure the absolute hygienic conditions of use of the same apparatus, the speculum 15 and/or the check valve 18 can present weakened breakage portions, which at the moment of their removal from the pipe member 17 break easily, thereby preventing the speculum or the check valve from being re-used for a further lavage treatment.

The extension pipe member 17 can be permanently secured to the pipe fitting 14, taking care to clean it each time after use.

The pipe member 17 preferably may be connected to the pipe fitting 14 in an easily removable way, for example by a self-coupling connector, or by means of a threaded nut, a snap coupling or in any other suitable way.

To prevent the seat 11 from shifting in relation to the bowl 10 when the patient sits or rests upon the seat, with the danger of suffering any injury at the time of self-insertion of the speculum 15, or during use, the seat 11 has been provided, at two or more points of its internal edge, with a slot for the insertion of a stop member 11' which extends downwards against the internal edge of the toilet bowl 10.

In the case of Fig. 1, the feed duct 13 for the lavage fluid consists of a separate tubular duct, fastened at the rear side of the seat 11 by means appropriate brackets, through a pair of hinges designed to enable the seat 11 and the lid 12 to be raised; furthermore, the pipe fitting 14 extends towards the toilet bowl 10, through a central slit provided in the rear side of the same seat.

The feed duct 13 for the lavage fluid opens out at both sides of the seat 11 to allow a connection to a pressurized source of lavage fluid, for example to a pipe network for sanitary water, according to the requirements.

In particular, one end of the feed duct 13 can be connected to a water network or to a pipe network for sanitary water, by means of a pressure regulator or pressure control valve 20 and a flow control valve 21 for controlling and adjusting the flow rate, and by a flexible hose 22.

The flow control valve 21 for example can be of the ball type, having one or more settable positions so as to control the flow rate of fluid fed to the injection speculum, thereby preventing the injection of strong jets of fluid which could injury or damage the intestine.

For this purpose, and in order to further reduce any possible risk, for example due to sudden pressure changes in the lavage fluid, one branch pipe or an end of the feed duct 13, opposite the one for the connection to the lavage fluid source, can be provided with an additional safety valving device comprising a maximum pressure valve 23, which can be set to open automatically whenever the pressure of the fluid exceeds a value considered as dangerous, for example ranging from 40 to 50 millibars (4000-5000 Pa).

In order to prevent solid particles of sand or lime entrained by the lavage fluid from blocking the pressure control valve 20, the maximum pressure valve 23, and the one-way valve 16, or occluding the injection holes in the speculum 15, it is possible to provide a suitable filter upstream of the pressure control valve 20 or the flow control valve 21.

The injection speculum 15 can be shaped in any way; for example it may present at its front end a slightly rounded head and/or a slightly tapered shape to facilitate its self-insertion into the anal portion of the intestine by the simple sitting on the seat 11.

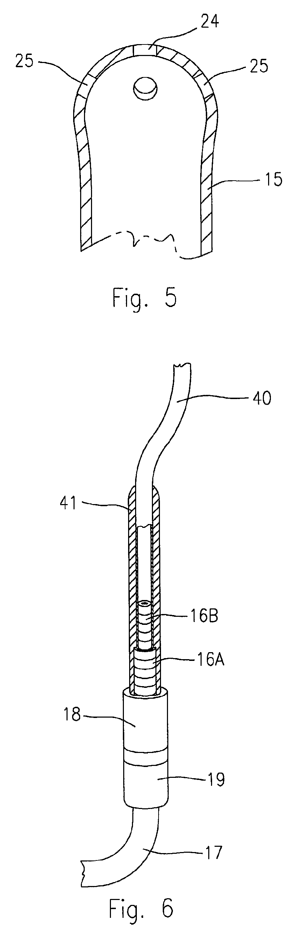

Moreover, the front end of the speculum 15 can present a central injection hole 24 and/or side injection holes 25, appropriately slanting upwards, as shown in the enlarged detail of Fig. 5.

The intestinal lavage can be carried out using only water at a suitable temperature, for example by connecting the apparatus to the water pipe network of hot and cold sanitary water by means of a suitable mixer.

However, it is preferable, or necessary in many cases, for the lavage fluid to be suitably mixed with a curative substance.

This is made possible by means of the apparatus according to this invention, by providing downstream the pressure control valve 20, a three-way connecting union 26, and a one-way check valve 27 which is automatically opened by the insertion of a container 28 for the curative substance, which in this way mixes by simply dropping into the lavage fluid while it is fed to the injection speculum 15.

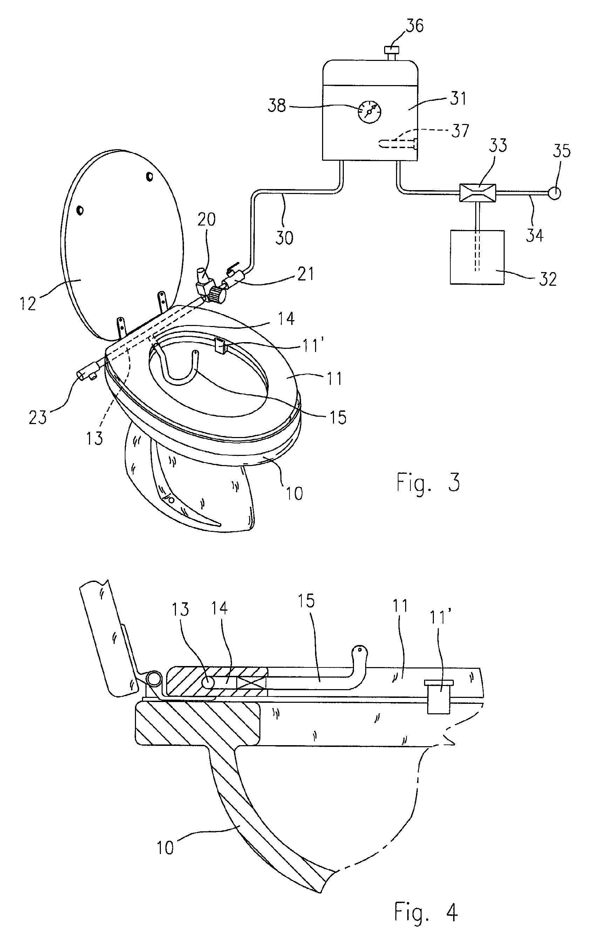

Fig. 3 of the accompanying drawings shows a second embodiment of the apparatus according to the invention.

In Fig. 3 the same reference numbers of Fig. 1 have been used to indicate similar or equivalent parts.

In Fig. 3, reference number 10 again has been used to indicate the toilet bowl, reference 11 the seat, reference 12 the lid and reference 13 the feed duct for the lavage fluid.

The solution of Fig. 3 differs from that of Fig. 1 in that the feed duct 13 for the lavage fluid, with the connecting pipe fitting 14, is embedded or built in directly into the rear side of the seat 11; moreover, as a further possible embodiment, the pipe 17 has been eliminated since the speculum 15 has been appropriately shaped to be press fitted, screwed or connected by any other releasable connection system, directly onto the pipe fitting 14 of the feed duct 13.

The speculum 15 may be shaped in any way, and provided with a one-way check valve. In the case shown, the speculum 15 presents an initial portion which is disengageably connected to the connecting pipe fitting 14, an intermediate V shaped portion which extends downwards inside the toilet bowl 10, and an end linear portion designed to penetrate the anal portion of the intestine, which extends upwards, beyond the seat 11, as shown.

Still in Fig. 3, reference numbers 20 and 21 have been used to indicate the pressure control valve and the flow control valve for the intestinal lavage fluid, and 23 the overpressure valve.

Unlike the previous case, according to the example of Fig. 3 the duct 13 is connected, by means of a pipe 30, to a tank 31 for containing the lavage fluid, in which a curative substance may be dissolved and which is automatically sucked from a container 32, for example by means of a Venturi tube 33 or other suitable device in the piping 34 which connects the tank 31 to a pressurised fluid source 35, for example to the water network or to a sanitary water supply system.

The tank 31 can comprise an air venting valve 36 and an electric heating element 37 for heating the water, controlled by an adjustable thermostat 38.

Fig. 4 shows, by way of example, the use of a differently shaped speculum; in particular, the speculum 15 presents a rectilinear portion having at its front end an upwardly oriented tip which extends beyond the seat 11.

According to a further feature of the invention, in the case of the solution of Fig. 1, by providing an appropriate joint system, it is possible to turn on the connecting pipe fitting 14 with the pipe member 17 sideways and upwards against the lid 12 in a condition of non-use.

Both the solution of Fig. 1 and the solution of Figg. 3 and 4 prove to be advantageous in that, by simply removing the speculum 15 and/or the pipe member 17, or by removing the speculum 15 and turning the connecting pipe fitting 14 with the pipe member 17, it is possible to restore the bowl 10 and the seat 11 for a normal use.

In the case of Figg. 1-5, it has been shown the use of a rigid speculum, of limited length, designed to partially penetrate the anal portion of the intestine, for example by approximately 5-6 cm, which is sufficient to allow the cleansing and lavage of the sigma colon, the rectum and the anus, efficiently and directly, and wholly independently.

In fact, due to its limited dimensions, the rigid speculum penetrates the intestine by a simple self-insertion at the sitting, with the utmost ease.

Since the terminal portion of the intestine, more correctly referred to as "rectum", presents a length of approximately 13-15 cm, and can undergo extensive dilation of the sphincter, and since the speculum has a diameter of approximately 10 mm, owing to the arise of a peristaltic stimulus created by the water injected into the intestine, a natural dilation of the sphincter occurs which permits easy outflow of the faecal material, without the speculum constituting a hindrance.

Consequently, contrary to the conventional equipments for this type of treatment, in which the peristaltic movement occurs with a strong stimulus and with a quite violent ejection of the faecal material, the apparatus according to this invention permits a much more gentle ejection action, in that it enables a greater control of the stimulus, and consequently a more thorough intestinal cleansing action.

As mentioned previously, university studies and clinical trials have proved that the most of tumours occur in the area of the colon; of these, 75% develop in the rectum, the sigma colon and the descending colon. The remaining 25% are found in the transverse colon and in the ascending colon.

It is obvious therefore that an intestinal lavage apparatus according to this invention which makes use of a rigid speculum, allows a defence in the prevention of a great majority of the tumours which develop in the sigma colon and in the descending colon.

Nevertheless, the possibility of using soft probes of suitable length, in association with the apparatus according to the invention, makes it possible to carry out deep lavages of the intestine, reaching as far as the descending colon and beyond, without causing trauma or damages.

However, in the event of a strong peristaltic stimulus, a soft probe or of the flexible type necessary for following the internal ansae of the intestine, could slip out, causing considerable inconvenience.

In order to solve this problem, according to another feature of the invention, a particular speculum has been provided, comprising a soft or flexible probe or tubular element 40 designed to penetrate deep into the intestine, and a rigid tubular support member 41 coaxially extending from an end of the tubular element of the probe for a length sufficient to partially penetrate the anal portion of the intestine, in a wholly similar way to the rigid speculum of the preceding figures.

A speculum of this kind is shown by way of example in Fig. 6, in which the fore end of the connecting pipe 17 is provided with the check valve 18 and with a double pipe fitting 16A and 16B for connection of the support member 41 and probe 40.

In particular, as shown in Fig. 6, the flexible probe 40, partially shown, is fitted onto the pipe fitting 16B of smaller diameter, while the rigid tubular support member 41, through which the soft probe 40 has been passed, is fitted onto the pipe fitting 16A of larger diameter; since the tubular support member 41 must be of such length as to penetrate the anal portion of the intestine, it is obvious that in this way the soft probe 40 is firmly held in the intestine, thereby preventing it from accidentally slipping out.

The apparatus and the use of a rigid speculum, besides for a lavage and a preventive cure against several intestinal pathologies, can also be useful for further applications, for example for preventive treatment against haemorrhoids, or for vaginal lavages.

In the case of haemorrhoids, it is known that they occur following exertion made by the sphincter in conditions of constipation. For this type of disorder, proctologists recommend the use of curative products and a diet which softens and facilitates the expulsion of the faecal residues.

The problem of softening the faecal matter can be easily overcome and solved with the apparatus according to this invention, in place of or in combination with the cures suggested by the doctor.

Conversely, in the case of vaginal lavages the apparatus according to this invention proves to be particularly effective in the treatment of candida albicans which, as is known, constitutes one of the more difficult and resistant pathologies to eradicate.

The apparatus according to this invention can consequently be of great help also in the problems set forth above, in that its practicality of use enables to immediately intervene, as soon as the disorder appears, thereby preventing possible bacterial proliferation.

As mentioned, the apparatus can be easily installed on any toilet bowl and can also be equipped with appropriate devices and safety appliances to prevent bacterial contamination upstream of the injection speculum, and violent jets which could damage the intestine.

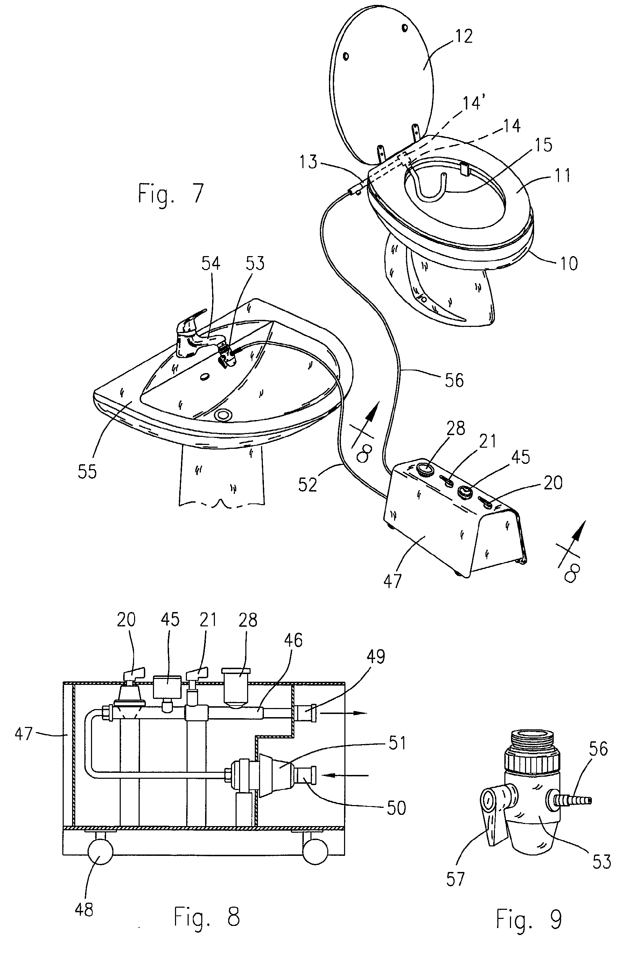

A further embodiment of an apparatus according to the invention is shown in Figg. 7 to 9 of the drawings. This solution differs from the previous ones, in that the hydraulic components such as the flow control valve 21, the pressure control valve 20, the container 28 for the curative substance and a pressure gauge 45 have been assembled on a manifold 46 inside a protective housing 47 provided with castor wheels 48.

The manifold 46 is directly connected to an inlet pipe fitting 49, and indirectly to an outlet pipe fitting 50 by means of a filter 51.

As shown in Figg. 7 and 8, the pipe fitting 49 is connected by a hose 52 and an auxiliary back-pressure control valve 53 to the spout of a mixer 54, such a mixer for hot/cold water of wash basin, lavatory and the like; at the same time the outlet pipe fitting 54 is connected to the pipe 13 of the seat 11, by a hose 56; a check valve 14' is provided in the pipe fitting 14 for the speculum 15.

More precisely as shown in Fig. 9, the hose 52 is connected to a pipe fitting 56 on a side of the back-pressure control valve 53 which may be manually actuated by a control lever 57 to allow the required flow of water for the lavage apparatus and at the same time a required back-pressure to actuate an usual gasheater to supply hot water.

From what has been described and shown in the accompanying drawings, it will be clear that what is provided is an apparatus for independent use, for intestinal lavages and for other similar applications, with a self-insertable speculum or probe, which in addition of being extremely simple, presents numerous advantages such as quick and easy use, a gentle and non-violent cleansing action, as well as, thanks to the use of a soft speculum allows to carry out deep lavages without any inconvenience. In all cases it is possible to carry out a deep cleansing as well as a preventive curative action against numerous intestinal pathologies.

The apparatus is also absolutely hygienic thanks to the use of a disposable type speculum, with the possibility of mixing medicinal products, according to need.

Consequently, what has been described with reference to the accompanying drawings, has been given purely by way of example to illustrate the general features of the apparatus according to the invention; other modifications or variations may be made without departing from the scope of the claims.

## Claims

1. Apparatus for intestinal lavages and the like, particularly suitable for use on a toilet bowl (10), **characterized by** comprising:
- a shaped seat (11) for a toilet bowl (10), having a rear side and an inner edge;
- a feed duct (13) for feeding a lavage fluid, said feeding duct (13) extending at the rear side of the seat (11), said feeding duct (13) comprising an end fitting (14) at the rear side of the seat (11);
- a disposable injection speculum (15), or probe, for injecting a lavage fluid, removably connected to the end fitting (14) of the feed duct (13), said speculum (15) having a portion which extends upwards from the inside of the toilet bowl (10), for a length sufficient to penetrate the anal tract of the intestine;
- manually actuable flow control valve (21) for adjusting the flow rate and for connection to a lavage fluid source; and
- manually actuable pressure control valve (20) for controlling the pressure of the lavage fluid fed to the injection speculum (15).

2. Apparatus for intestinal treatment, according to claim 1, **characterized by** comprising means (13;31,32,33) for containing and delivering a curative substance into the lavage fluid fed to the injection speculum (15).

3. Apparatus for intestinal treatment, according to claim 2, **characterized in that** said means (13;31,32,33) for delivering the curative substance comprise a container (28) for drop feeding the curative substance, and a one-way check valve (27) branched off from the feed duct (13) for the lavage fluid.

4. Apparatus for intestinal treatment, according to claim 1, **characterized by** comprising a maximum pressure valve (23) branched off from the feed duct (13).

5. Apparatus for intestinal treatment, according to claim 1, **characterized by** comprising a one-way check valve (14',18), upstream of the injection speculum (15).

6. Apparatus for intestinal treatment, according to claim 1, **characterized in that** the injection speculum (15) is removably connected to a V-shaped extension pipe (17).

7. Apparatus for intestinal treatment, according to claim 6, **characterized in that** the extension pipe (17) is removably connected to a pipe fitting (14) of the feed duct (13).

8. Apparatus for intestinal treatment, according to claim 1, **characterized in that** the injection speculum (15) is provided with a weakened breakage portion to prevent reuse.

9. Apparatus for intestinal treatment, according to claim 1, **characterized in that** the injection speculum (15) comprises a flexible probe (40) and a rigid tubular supporting member (41) coaxially arranged at an end of the flexible probe (40).

10. Apparatus for intestinal treatment, according to claim 9, **characterized in that** said flexible probe (40) and/or tubular supporting member (41) present weakened breakage points to prevent their reuse.

11. Apparatus for intestinal treatment, according to claim 1 or 9, **characterized in that** the injection speculum (15;40,41) is connected to the pipe fitting (14) of the feed duct (13), or to its tubular extension (17), by means of a pivotable joint (19).

12. Apparatus for intestinal treatment, according to claim 1, **characterized in that** the lavage fluid source comprises a storage tank (31).

13. Apparatus for intestinal treatment, according to claim 12, **characterized by** comprising means for mixing a curative substance, connected to said storage tank (31).

14. Apparatus for intestinal treatment, according to claim 12, **characterized in that** the storage tank (31) comprises electric heating means (37) and a control thermostat (38).

15. Apparatus for intestinal treatment, according to claim 1, **characterized in that** the seat (11) comprises stop means (11') resting against the internal edge of the toilet bowl (10).

16. Apparatus for intestinal treatment, according to claim 1, **characterized in that** the feed duct (13) and the pipe fitting (14) for the lavage fluid are embedded into the seat (11).

17. Apparatus for intestinal treatment, according to claim 6, **characterized in that** the pipe fitting (14) and the extension pipe (17) are tiltably arranged on the seat (11).

18. Apparatus for intestinal treatment, according to claim 1, **characterized in that** the speculum (15) comprises a plurality of differently oriented injection holes (24,25) on its fore end.

19. Apparatus for intestinal treatment, according to claim 1, **characterized in that** the flow control valve (21) and the pressure control valve (20), as well as a pressure gauge (45) and a container (28) for the curative substance are provided inside a movable housing (47).
